# EUROPEAN PATENT SPECIFICATION

(11) **EP 1 799 277 B1**
(45) Date of publication and mention of the grant of the patent: **07.11.2012**
(21) Application number: 04775616.8
(22) Date of filing: 14.09.2004
(51) Int. Cl.: A61L 27/12, A61L 27/24, A61L 27/44, A61L 27/56

(54) **POROUS BIOMATERIAL-FILLER COMPOSITE AND A METHOD FOR MAKING THE SAME**
PORÖSER VERBUNDWERKSTOFF AUS BIOLOGISCHEM MATERIAL UND FÜLLSTOFF UND VERFAHREN ZU SEINER HERSTELLUNG
COMPOSITE DE MATIERE DE CHARGE BIOLOGIQUE POREUSE ET PROCEDE DE FABRICATION CORRESPONDANT

(43) Date of publication of application: 27.06.2007
(73) Proprietor: Agency for Science, Technology and Research, Singapore 138668 (SG)
(72) Inventor: YING, Jackie, Y., Singapore 508056 (SG); PEK, Shona, Singapore 229978 (SG); GAO, Shujun, Singapore 120712 (SG); MOHAMED SHARIFF MOHAMED ARSHAD, Singapore 138669 (SG); MAO, Pei Lin, Singapore 689048 (SG)
(74) Representative: Clegg, Richard Ian
(86) International application number: PCT/SG2004/000294
(87) International publication number: WO 2006/031196

(56) References cited:
- WO-A1-98/46164
- WO-A1-98/54089
- WO-A1-03/092760
- US-A- 5 034 352
- US-A- 5 264 214
- US-A1- 2003 232 071
- US-A1- 2004 091 547
- US-A1- 2004 138 758
- DATABASE WPI Week 200377, Derwent Publications Ltd., London, GB; AN 2003-813856, XP008107568 & CN 1 403 165 A (UNIVERSITY SOUTHEAST) 19 March 2003
- LU W.W. ET AL: 'Controlled porosity hydroxyapatite ceramics as spine cage: fabrication and properties evaluation' JOURNAL OF MATERIAL SCIENCE, MATERIALS IN MEDICINE vol. 14, 2003, pages 1039 - 1046, XP008115607
- LIU L. ET AL: 'Preparation and characterization of collagen-hydroxyapatite composite used for bone tissue engineering scaffold' ARTIF. CELLS, BLOOD SUBSTITUT. AND BIOTECHNOLOGY vol. 31, no. 4, 2003, pages 435 - 448, XP008107602
- ZHAO F. ET AL: 'Preparation and histological evaluation of biomimetic three-dimensional hydroxyapatite/chitosan-gelatin network composite scaffolds' BIOMATERIALS vol. 23, 2002, pages 3227 - 3234, XP008107541
- DATABASE MEDLINE [Online] YUAN Y. ET AL: 'Synthesis of nanometer hydroxyapatite by sol-gel method', XP008138548 Database accession no. (NLM12905788) & ZHONGGUO YI XUE KE YUAN XUE BAO vol. 24, no. 2, April 2002, pages 129 - 133
- BEZZI G. ET AL: 'A novel sol-gel technique for hydroxyapatite preparation' MATERIALS CHEMISTRY AND PHYSICS vol. 78, 2003, pages 816 - 824, XP008107542
- PARK J.-C. ET AL: 'A bone replaceable artificial bone substitute: morphological and physiochemical characterizations' YONSEI MED. J. vol. 41, no. 4, 2000, pages 468 - 476, XP002964719

## Description

### Technical Field

The present invention relates to a porous biomaterial-filler composite, a method of preparation, and uses thereof. In one particular embodiment, the present invention relates to a collagen-inorganic scaffold.

### Background of the Invention

30 to 35% of bone is composed of organic material (on a dry weight basis Of this amount, about 95% is collagen. The remaining organic substances are chondroitin sulfate, keratin sulfate and phospholipids. 65 to 70% of bone is composed of inorganic substances. Almost all of these inorganic substances are composed of hydroxyapatite

When large amounts of lost bone need replacement, this is usually achieved by a variety of grafts or permanent alloy implants, Such grafting and implants are sometimes not desirable as they may not have sufficient strength to support an active lifestyle or sufficient bioactivity to promote cell attachment and proliferation. Many implants used are also not resorbable by natural tissue and cannot be tunable with respect to their mechanical properties or degradation rates.

Collagen has a low immunogenicity, is bioabsorbable and is a naturally occurring structural protein to which cells can attach, interact with and degrade. Collagen sponges and foams have been used as hemostatic agents, as scaffolds for tissue repair and as a support for cell growth. To date, however, no implant or collagen sponge has had the same or similar properties to that of natural bone.

### Object of the Invention

It is an object of the present invention, at least in preferred embodiments, to overcome or substantially ameliorate at least one of the above disadvantages.

### Summary of the Invention

According to a first aspect of the present invention, there is provided a porous biomaterial-filler comprising biomaterial interspersed with a filler, wherein the dry weight ratio of the biomaterial to the filler is in the range of 1:3 to 2:1, and wherein the filler is prepared by a sol-gel method to form nano-sized grains in the filler, and wherein the composite includes pores having a size greater than 100 microns, pores having a size between 30 and 50 microns and nanometer sized pores.

Also described herein is a porous biomaterial-filler composite wherein the composite comprises biomaterial interspersed with a filler and wherein the dry weight ratio of biomaterial to filler is in the range 1:3 to 2:1.

In one embodiment, the composite comprises by dry weight 25 to 50wt% biomaterial and 35 to 75wt% filler.

According to an aspect of the present invention, there is provided a porous biomaterial-filler composite wherein the composite comprises biomaterial interspersed with a filler wherein the amount of biomaterial is at least 25wt%.

As described herein the composite may have a porosity close to that of bone.

Also described herein is a porous biomaterial-filler composite wherein the composite comprises biomaterial interspersed with a filler and wherein the composite filler has been prepared using a sol-gel method.

According to another aspect of the present invention, there is provided a method of preparing a porous biomaterial-filler composite, said composite having a dry weight ratio of biomaterial to filler in the range of 1:3 to 2:1 and having (a) pores having a size greater than 100 microns; (b) pores having a size between 30 and 50 microns; and (c) smaller nanometer size pores, said method comprising
combining biomaterial, a liquid and a filler to form a mixture, said filler prepared by a sol-gel method to form nano-sized grains in the filler,
homogenizing the mixture to form a slurry, and
freeze-drying the slurry to form a porous biomaterial-filler composite.

According to another aspect of the present invention, there is provided a method of preparing a porous biomaterial-filler composite, said composite having a dry weight ratio of biomaterial to filler in the range of 1:3 to 2:1 and having (a) pores having a size greater than 100 microns; (b) pores having a size between 30 and 50 microns; and (c) smaller nanometer size pores, said method comprising
combining biomaterial and a liquid to form a mixture,
homogenizing the mixture to form a slurry,
adding a filler to the slurry, said filler prepared by a sol-gel method to form nano-sized grains in the filler;
further homogenizing the slurry; and
freeze-drying the slurry to form a porous biomaterial-filler composite.

According to another aspect of the present invention, there is provided a method of preparing a porous biomaterial-filler composite, said composite having a dry weight ratio of biomaterial to filler in the range of 1:3 to 2:1 and having (a) pores having a size greater than 100 microns; (b) pores having a size between 30 and 50 microns; and (c) smaller nanometer size pores, said method comprising
combining a filler and a liquid to form a mixture, said filler prepared by a sol-gel method to form nano-sized grains in the filler,
homogenizing the mixture to form a slurry,
adding a biomaterial to the slurry;
further homogenizing the slurry; and
freeze-drying the slurry to form a porous-biomaterial-filler composite.

The liquid may be water or any other liquid capable of forming a slurry. In one embodiment when the biomaterial is collagen, the liquid is an acid.

In some embodiments the liquid is an acid and the amount of biomaterial in the slurry is in the range of up to about 10g biomaterial per 100ml of up to a 1000mM acid.

### Definitions

The following definitions are intended as general definitions and should in no way limit the scope of the present invention to those terms alone, but are put forth for a better understanding of the following description.

Unless the context requires otherwise or specifically stated to the contrary, integers, steps, or elements of the invention recited herein as singular integers, steps or elements clearly encompass both singular and plural forms of the recited integers, steps or elements.

Throughout this specification, unless the context requires otherwise, the word "comprise", or variations such as "comprises" or "comprising", will be understood to imply the inclusion of a stated step or element or integer or group of steps or elements or integers, but not the exclusion of any other step or element or integer or group of elements or integers. Thus, in the context of this specification, the term "comprising" means "including principally, but not necessarily solely".

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations or any two or more of said steps or features.

In the context of this specification, the term "biomaterial" refers to any material which is suitable for introduction into a living organism such as a mammal including a human. The biomaterial is suitably non-toxic and bioabsorbable when introduced into a living organism and any degradation products of the biomaterial are also suitably non-toxic to the organism. The biomaterial may be derived from an organism, or may be a synthetic variant.

### Brief Description of the Drawings

A preferred form of the present invention will now be described by way of example with reference to the accompanying drawings wherein:
Figure 1 is a schematic diagram of a suitable method of preparing a composite material (bone scaffold) of the present invention;
Figure 2 is a set of Scanning Electron Micrographs of collagen suitable for use in the present invention;
Figure 3 is a set of Scanning Electron Micrographs of hydroxyapatite suitable for use in the present invention;
Figures 4A, 4B, 4C and 4D are graphs of stress with respect to strain of various porous scaffolds in accordance with the present invention;
Figure 5 is a set of Scanning Electron Micrographs of trabecular bone at various resolutions;
Figure 6 is a set of Scanning Electron Micrographs of a collagen-inorganic scaffold in accordance with one embodiment of the present invention at various resolutions;
Figure 7 is a set of Scanning Electron Micrographs of a collagen-inorganic scaffold in accordance with another embodiment of the present invention at various resolutions;
Figure 8 is a set of Scanning Electron Micrographs at various resolutions and an XRD spectrum of a collagen-inorganic scaffold in accordance with another embodiment of the present invention;
Figure 9 is a graph showing XRD patterns for two collagen-inorganic scaffolds in accordance with the invention compared with that of natural bone and carbonated hydroxyapatite;
Figure 10 is a gaph showing XRD patterns for four collagen-inorganic scaffolds in accordance with the invention compared with that of calcium carbonate and brushite;
Figure 11 is the FTIR spectrum of various starting materials, collagen-inorganic scaffolds in accordance with one embodiment of the present invention and natural trabecular bone.
Figure 12 is a set of Scanning Electron Micrographs of MC3T3 (mouse osteoblasts) cells cultured for 1 week on a scaffold in accordance with one embodiment of the present invention;
Figure 13 is a photograph of a 60 day ectopic implantation of a scaffold in accordance with the present invention into a SCID mouse;
Figure 14 is a micrograph of implanted scaffold tissue in accordance with the present invention stained with hematoxylin and eosin after 8 days;
Figure 15 is a micrograph of implanted scaffold tissue in accordance with the present invention stained with hematoxylin and eosin after 30 days;
Figure 16 is a micrograph of implanted scaffold tissue in accordance with the present invention stained with von Kossa after 8 days;
Figure 17 is a micrograph of implanted scaffold tissue in accordance with the present invention stained with von Kossa after 30 days;
Figure 18 is a set of photographs of a Wistar rat femur having a scaffold in accordance with the present invention implanted for six months; and
Figures 19A, 19B and 19C are X-rays of Wistar rat femur having a scaffold in accordance with the present invention at 5 months post-implantation (Figure 19A) and that of Wistar rat femur having a commercial scaffold (Figure 19B) or no scaffold at all (Figure 19C).

### Detailed Description of Preferred Embodiments

There is provided herein a porous biomaterial-filler composite, the composite comprising biomaterial interspersed with a filler. The present invention provides in one embodiment, a composite comprising a biomaterial phase and inorganic filler interspersed therein. Suitably a polymer phase with an inorganic filler interspersed therein. The biomaterial and the interspersed filler may be chemically bonded to each other.

In one embodiment the composite has a porosity that closely matches that of natural bone. It is desirable in the composite of the present invention in some embodiments to match natural bone as closely as possible, structurally, chemically and mechanically so that a body, in which the composite is implanted can recognize and remodel the composite similarly to natural bone.

In one embodiment the composite has pores having a size greater than 100 microns, pores having a size between 30 and 50 microns and small nanometer size pores. The small nanometer-sized pores may be made by voids between filler particles. Such porosity may closely match the actual structure of bone, the large pores enabling osteoblast migration, the medium pores enabling transport of blood/proteins/fluids and the small pores providing traction for better cell attachment.

In one embodiment, the composite contains at least 25 wt% biomaterial. The filler may be up to 75wt%. In another embedment the composite contains 25 to 50wt% (dry weight) biomaterial with the amount of filler being between 35 to 75wt% (dry weight). The amounts and biomaterial and filler used may be tailored to have an organic:inorganic ratio to match the composition of natural bone. For example 30 to 35wt% collagen and 65-70% hydroxyapatite.

In one embodiment, the filler may have previously been prepared by a sol-gel method. Use of a filler prepared in this way may enable pores of nanometer porosity.

There is also provided herein in one embodiment a method of preparing a porous biomaterial-filler composite comprising
combining biomaterial, a liquid and a filler to form a mixture,
homogenizing the mixture to form a slurry, and
freeze-drying the slurry to form a porous biomaterial-filler composite.

There is also provided herein in another embodiment a method of preparing a porous biomaterial-filler composite comprising
combining biomaterial and a liquid to form a mixture;
homogenizing the mixture to form a slurry,
adding a filler to the slurry;
further homogenizing the slurry; and
freeze-drying the slurry to form a porous biomaterial-filler composite.

There is also provided herein in another embodiment a method of preparing a porous biomaterial-filler composite comprising
combining filler and a liquid to form a mixture,
homogenizing the mixture to form a slurry,
adding a biomaterial to the slurry;
further homogenizing the slurry; and
freeze-drying the slurry to form a porous biomaterial-filler composite.

In one embodiment, the composite contains at least 25wt% biomaterial. In one embodiment the liquid is an acid and the amount of biomaterial in the slurry is in the range of up to about 10g biomaterial per 100ml of an up to 1000mM acid. For example 0.6 to 6g biomaterial such as collagen per 100ml of 50 to 500mM acid such as phosphoric acid (6mg/ml to 60mg/ml) results in a composite having different mechanical properties and microstructure.

Examples of some sample compositions as starting materials include:
100 ml of 50 mM phosphoric acid + 2 g collagen + 7.5 g CAP (carbonated apatite).
100 ml of 50 mM phosphoric acid + 2 g collagen + 12 g HAP (hydroxyapatite)
100 ml of 100 mM phosphoric acid + 2 g collagen + 10 g CaCO₃ (calcium carbonate)
100 ml of 50 mM phosphoric acid + 4 g collagen + 7.5 g CAP (carbonated apatite)
100 ml of 50 mM phosphoric acid + 4 g collagen + 12 g HAP (hydroxyapatite)
100 ml of 500 mM phosphoric acid + 4 g collagen + 10 g CaCO₃ (calcium carbonate).

In one embodiment, the biomaterial is a material extracted from biological tissue, including for example, fetal tissue, skin/dermis, muscle or connective tissue, including bone, tendon, ligament or cartilage. In one embodiment the biomaterial is a biopolymer. A biopolymer is suitably a naturally occurring polymeric substance in a biological system or organism. Biopolymers can also suitably be man-made polymers prepared by manipulation of a naturally occurring biopolymer.

Any biomaterial that may be formed into a stable solid structure at body temperature, for example dried into a film, solidified from a melt, cross-linked into a gel, freeze-dried into a foam may be used in the present invention.

In one embodiment the biomaterial is selected from one or more of proteins, peptides, polysaccharides or other organic substances. For example the biomaterial may be selected from one or more of extracellular matrix proteins such as fibronectin, laminin, vitronectin, tenascin, entactin, thrombospondin, elastin, gelatin, collagen, fibrillen, merosin, anchorin, chondronectin, link protein, bone sialoprotein, osteocalcin, osteopontin, epinectin, hyaluronectin, undulin, epiligrin and kalinin, proteoglycans such as decorin, dermatin sulfate proteoglycans, keratin, keratin sulfate proteoglycans, aggrecan, chondroitin sulfate proteoglycans, heparin sulfate proteoglycans, biglycan, syndecan, perlecan, serglycin, glycosaminoglycans such as heparin sulfate, chondroitin sulfate, dermatin sulfate, keratin sulfate or hyaluronic acid, polysaccharides such as heparin, dextran sulfate, chitin, alginic acid, pectin or xylan, polyvinyl alcohol, cytokines, glycosides, glycoproteins, polypyrroles, albumin, fibrinogen, or a phospholipid.

In one embodiment the biomaterial is collagen. In one further embodiment the collagen is one or more selected from the group consisting of collagen Type I, collagen Type II, collagen Type III, collagen Type IV, collagen Type V, collagen type VI, collagen Type VII, collagen Type VIII, collagen Type IX, collagen Type X, collagen Type XI, collagen Type XII, collagen Type XIII, collagen Type XIV, or mixtures thereof. In one embodiment the collagen is Type 1 collagen.

The biomaterial may be extracted from a source by means of acid extraction, salt extraction, enzyme/pepsin extraction or a combination thereof or by some other means such as mechanical extraction for example by grinding. The biomaterial may be prepared by acid extraction followed by precipitating the biomaterial (such as collagen) with sodium chloride and resolubilizing the biomaterial (such as collagen) in a medium having an acidic pH.

Sources of biomaterials include both land and marine vertebrates and invertebrates, for example a mammal, marsupial, a human, a non-human primate, murine, bovine, ovine, equine, caprine, leporine, avian, feline, porcine or canine. In one embodiment the biomaterial is sourced from a mammal or marsupial such as a human, pig, cow, sheep, deer, goat, horse, donkey, hare, rat, mouse, rabbit, kangaroo, wallaby or camel.

In one embodiment, the composite may be formed into a foam, gel or other construct including fibres. In one embodiment the composite is in the form of a porous foam. The foam may include a network of communicating microcompartments with biomaterial molecules and/or filaments interspersed throughout. In an alternative embodiment, the composite may be in the form of biomaterial filaments or fibres having an inorganic filler interspersed therein. In one embodiment the composite is in the form of a cross-linked spongy foam. In another embodiment the composite is in the form of a stiff foam. In one embodiment, the composite may be in the form of a scaffold. A scaffold is a substratum which may be used for anchoring cells.

In one embodiment the filler comprises one or more inorganic compounds. In one embodiment the filler is selected to improve the compressive modulus of the composite. In one embodiment, the inorganic filler comprises calcium carbonate or a calcium-containing salt. In another embodiment, the inorganic filler comprises calcium phosphate, such as an apatite or substituted apatite. In a further embodiment the filler comprises a combination of calcium carbonate and a calcium phosphate such as an apatite. In one embodiment, the calcium phosphate is brushite, tricalcium phosphate, octacalcium phosphate. In various embodiments an apatite is selected from hydroxyapatite (HAP), fluoroapatite (FAP), carbonated apatite (CAP) or zirconium hydroxyapatite (ZrHAP). Apatites containing dopants and additives or substituted apatites may also be used. The filler may suitably be in powder form. In one embodiment the filler is made using a sol-gel method. The sol-gel method is such as described in "Nanostructure Processing of Hydroxyapatite-based Bioceramics", ES Ahn, NJ Gleason, A. Nakahira, JY Ying Nano Letters 2001 1(3) p149-153. Producing filler by use of a sol-gel method may enable nanometer-sized grains to be formed and which in the final composite may form small nanometer-sized pores from the voids between the apatite particles. In one embodiment a carbonated apatite prepared by the sol-gel method having a grain size of between 8 to 20nm (for example grains of 8, 9, 10, 11, 12, 13, 14, 15, 16, 17, 18, 19 or 20nm) or a hydroxyapatite prepared by the sol-gel method having a grain size of between 40 to 60nm (for example 40, 41, 42, 43, 44, 45, 46, 47, 48, 49, 50, 51, 52, 53, 54, 55 56, 57, 58, 59 or 60nm) may be used. The grain size of the filler may range from 5nm up to micron range sizes. When the filler is an apatite, the grain size of the filler used may range from 5 to 100nm.

In another embodiment, the filler is demineralised bone, bone powder, bone morphogenetic protein, calcium sulfate, autologous bone, beads of wax, beads of gelatin, beads of agarose; resorbable polymers or a mixture thereof. These may be used either alone or together and may be used in addition or in place of the apatites and calcium containing compounds.

For preparation of the composite, in one embodiment the liquid is any liquid or fluid capable of forming a slurry. For example the liquid may be water, an organic solvent, an acid, a base or a surfactant. In one embodiment the liquid may be an acid. When the biomaterial is collagen, the liquid may be an acid so as to enable proper dispersion of the collagen. The liquid may include salts or other additives. In a further embodiment the liquid is an inorganic acid. In another embodiment the liquid is an organic acid. For example the liquid may be phosphoric acid. Other acids which may be used include acetic acid, lactic acid, formic acid, tartaric acid, sorbic acid, sulfuric acid, hydrochloric acid, phosphoric acid, ascorbic acid, propanoic acid, triflic acid, trifluoroacetic acids or other acid. Where the composite involves incorporation of calcium carbonate, it is desirable to use phosphoric acid since the reaction between calcium carbonate and an acid other that phosphoric acid may not produce desirable calcium phosphates.

The acid used may have a concentration of up to 1000mM, for example about 1 mM up to about 500mM. As further examples 1mM, 5mM, 10mM, 20mM, 30mM, 40mM, 50mM, 60mM, 70mM, 80mM, 90mM, 100mM, 150mM, 200mM, 300mM, 400mM, 500mM, 600mM, 700mM, 800mM, 900mM or 1000mM acid. In one embodiment the acid has a concentration of up to about 150mM. In another embodiment the acid has a concentration of up to about 100mM. Where the filler is calcium carbonate, suitably about 1mM to 500mM, for example 100mM phosphoric acid is used. Where the filler is an apatite, suitably about 1mM to about 500mM, for example 50mM phosphoric acid is used.

In one embodiment the liquid is phosphoric acid and the filler is calcium carbonate. In this embodiment, the phosphoric acid may react with the calcium carbonate to form calcium phosphates which may be precipitated onto the biomaterial.

In one embodiment, when the liquid is an acid, the slurry may include up to 10g of the biomaterial per 100ml of the acid, alternatively up to 9g, up to 8g, up to 7g, up to 6g, up to 5g, up to 4g, up to 3g, up to 2g, up to 1g or up to 0.5g biomaterial per 100ml of the acid. In one embodiment the slurry includes 0.6g biopolymer per 100ml of the acid. In another embodiment the slurry includes 2g biopolymer per 100ml of the liquid. By controlling the ratio of biomaterial to liquid it is possible to modify or control the mechanical properties and microstructure of the composite.

In one embodiment the amount of filler used is selected so that the ratio of biomaterial:powder is similar to that present in the biological system into which the composite is to be inserted. In one embodiment 5 to 15g of filler is used per 100ml of liquid. Alternatively 6g, 7g, 8g, 9g, 10g, 11g, 12g, 13g, 14g of filler are used per 100ml of liquid. In one embodiment a first composite/scaffold may be prepared comprising 100ml liquid, 0.6g collagen and filler in the range 5 to 15g to give a scaffold with a higher porosity due to the higher liquid:solid ratio. In another embodiment a second composite/scaffold may be prepared comprising 100ml liquid, 2g collagen and filler in the range 5 to 15g which may be useful for insertion as a replacement for bone as it is has lower porosity, is less brittle under compression and has a higher compressive modulus that the first mentioned scaffold.

In one embodiment the mixture is homogenized/dispersed in a mixer. In one embodiment the mixer is a vortex mixer. The mixture may be homogenized/dispersed for from 1 minute to 10 hours. In one embodiment the mixture may be homogenized/dispersed for from 10 minutes to 6 hours. For example, the mixture may be homogenized/dispersed for 1 hour. As further examples, the mixture may be homogenized/dispersed for 5, 10, 20, 30, 40, 50, 60 minutes, 1, 2, 3, 4, 5, 6, 7, 8, 9 or 10 hours. The slurry may be homogenized/dispersed at 1000 to 60000 rpm. In one embodiment the mixture is homogenized/dispersed at 6000 to 50000rpm, for example at about 14,000 rpm to form the slurry. Other examples of speeds of the mixer are 1000, 2000, 3000, 4000, 5000, 6000, 7000, 8000, 9000, 10000, 11000, 12000, 13000, 14000, 15000, 16000, 17000, 18000, 19000, 20000, 30000, 40000, 50000, 60000rpm. The mixture may be cooled, for example, in an ice bath during homogenization, for example at 4°C. Other examples of temperature for cooling include 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C or 30°C. In one embodiment the mixture is further subjected to sonication to improve homogeneous dispersion.

In accordance with the process of the present invention, the slurry is freeze-dried. By freeze-drying porosity is created in the composite. Porosity may facilitate cell attachment and mobility and may assist blood vessels to infiltrate to allow fluid transport through the scaffold. In one embodiment the pore size and porosity may be controlled by controlling the freezing rate and/or water content of the homogenized slurry prior to freeze-drying. In another embodiment pore size and porosity may be controlled by selection of suitable starting materials and their amounts. In embodiments where an acid such as phosphoric acid and a carbonate such as calcium carbonate are respectively used as liquid or filler, additional porosity may be achieved by production of carbon dioxide bubbles during the reaction between the acid and carbonate.

In one embodiment the composite is subjected to two freezing rates (-80°C or -20°C freezer). The freeze-drying protocol may be optimized to control the pore structure, for example, the number and size of pores.

In one embodiment the slurry from the ice bath which may be at 4°C is poured onto a tray and placed in a freezer held at temperature which may be in the range 0°C to -50°C, for example -5°C, -10°C, -15°C, -20°C, -25°C, -30°C, -35°C, -40°C, -45°C In one embodiment the slurry is poured in a tray and placed in an -20°C freezer and left to freeze for between 1 to 12 hours depending on the quantity of the mixture. For example the slurry may be frozen for 1, 2, 3, 4, 5, 6, 7, 8, 9, 10, 11 or 12 hours. The frozen mixture may be freeze-dried until a dry porous solid foam is obtained using the following evacuation and heating phases:

Evacuation phase: The freeze-dryer used may be set up so that the condenser temperature is suitably between 0°C and -105°C. For example between -40 and -75°C. The vacuum in the freeze-dryer used may be pulled until it is between 4.58 to 0.005 torr (0.61kPa- 0.00067kPa). For example between 0.15 to 0.035 torr (0.012 - 0.0047 kPa).

Heating phase: The frozen mixture may be brought from freezing temperature, for example -20°C into a room temperature environment while simultaneously subjecting to the abovementioned vacuum. This may enable the ice crystals to sublimate, leaving behind pores.

In one embodiment the slurry is held at a temperature of 0°C to 30°C, for example 5°C, 10°C, 15°C, 20°C, 25°C for from 0 to about 60 minutes, for example 10, 15, 20, 25, 30, 35, 40, 45, 50, 55 minutes. The temperature is then suitably reduced at a ramp rate of from <1 to 50°C/min, for example at a ramp rate of 1°, 5°, 10°, 15°, 20°, 25°, 30°, 35°, 40°, 45°C/min to a final temperature in the range from -5°C to -80°C, for example -10, -15, -20, -25, -30, -35, -40, -50, -60, -70°C. This final temperature is suitably held for from 5 minutes up to 12 hours or more. In one embodiment the freeze-dryer may suitably be set up so that the condenser temperature is between - 105°C to 0°C, for example between -40°C and -75°C. Vacuum may be pulled until it is between 4.58 torr (0.61kPa) and 0.005 torr (0.00067kPa), for example between 0.15 torr (0.12kPa) and 0.035 torr (0.0047kPa).

In one embodiment the composite is freeze-dried until substantially all water has been sublimated.

The composite produced may be further processed by the addition of for example coating materials so as to make the properties of the composite similar to that of natural borne. For example the biomaterial may be polymerized or the slurry may be treated with an enzyme such as lysyl oxidase. In another embodiment the biomaterial may be esterified, acylated, deaminated or blocked with a blocking agent. Additives may be added to the slurry. For example fibre reinforcement, polypeptides, glycoprotein antifreezes, pharmaceuticals, antibiotics, growth factors or bone morphogenetic protein may be added to the slurry.

In one embodiment the composite may be conditioned with cells. Suitable cells include, but are not limited to, epithelial cells such as keratinocytes, adipocytes, hepatocytes, neurons, glial cells, astrocytes, podocytes, mammary epithelial cells, islet cells, endothelial cells such as aortic, capillary and vein endothelial cells, and mesenchymal cells such as dermal fibroblasts, mesothelial cells, stem cells, osteoblasts, smooth muscle cells, striated muscle cells, ligament fibroblasts, tendon fibroblasts, chondrocytes or fibroblasts.

In one embodiment, the freeze-dried composite is cross-linked. Cross-linking may increase the compressive modulus of the composite and improve its resistance to degradation. Cross-linking may cause the composite to become more physically stable and insoluble in aqueous medium. In one embodiment the composite/scaffold degradation rate can be controlled by varying the extent of cross-linking in the composite such as described by Y.S. Pek et al. in Biomaterials 25 (3) (2004) p 473-482. For example the freeze-dried composite may be cross-linked so that it forms a stiff foam or a spongy foam or an intermediate between a stiff foam and spongy foam.

In one embodiment the freeze-dried product may be subjected to cross-linking. In another embodiment, the slurry may be cross-linked prior to freeze-drying, Cross-linking may be performed by a cross-linking method such as by amide cross-linking. Example 1 describes one method of amide cross-linking using EDC/NHS.

Other cross-linking methods or cross-linking agents may be used. For example, physical, chemical and enzymatic methods of cross-linking may be used. Cross-linking may be performed with acrylamides, diones, glutaraldehyde, acetaldehyde, formaldehyde or ribose. UV or other irradiation methods such as gamma irradiation, dehydrothermal methods may be used.

Compared with current scaffolds on the market, the scaffolds of the present invention have better mechanical properties and microstructural and chemical match to natural bone, and are more osteoinductive.

The composite/scaffold of the present invention has the advantage that it has sufficient mechanical strength for load bearing applications and sufficient bioactivity to promote attachment and proliferation. The material of the invention may be resorbable by natural tissue and tunable to mechanical properties and degradation rates. The material of the invention may have microporosity of the order of about 100 to 600 microns, for example = 200 microns. The material is biocompatible and capable of being resorbed and replaced by tissue. The composite/scaffold in one embodiment of the invention has porosity equivalent to that of bone.

The composite of the present invention can be used in methods of replacing or repairing bone by implanting the composite.

The composite/scaffolds are suitable for orthopedic or other load-bearing applications. The scaffold can be used as an osteoinductive load-bearing hard tissue implant and can be also used for other tissue engineering applications. The scaffolds can be used as a resorbable bone substantive to aid in healing of large fractures and bone loss. The scaffolds Have sufficient strength to support the daily activities of the host animal during recovery. They demonstrate sufficient bioavailabity *in vivo* for rapid cell attachment. The scaffold can be used in tissue repair or reconstruction enabling regeneration of replacement tissue, for dressings, as hemostatic agents or as a support for cell growth *in vivo* and *in vitro*. The scaffold can also be used as a carrier containing protein or drugs for delivery. The scaffold can be used as a model system for research or as prostheses or implants to replace damaged or diseased tissues or to provide scaffolds which when occupied by cells are remodeled to become functional tissues. The scaffold can be seeded with cells and can be seeded with cells of the same type as those of the tissue which the scaffold is used to repair, reconstruct or replace. The scaffold may also be seeded with stem cells. The scaffold can be used as a prosthesis or implant and can be used to replace tissue such as skin, nervous tissue, vascular tissue, cardiac tissue, pericardial tissue, muscle tissue, ocular tissue, periodontal tissue, connective tissue such as a bone, cartilage, tendon or ligament, organ tissue, liver tissue, glandular tissue, mammary tissue, adrenal tissue, urological tissue and digestive tissue. The scaffold can be used as an implant which can be introduced or grafted into a suitable recipient such as a mammal including a human. The scaffold can also be used as a dressing such as a skin dressing or for drug delivery.

The composite/scaffold of the present invention may be applied topically, subcutaneously, intraperitoneally or intramuscularly.

### Example A

The following example is a description of a method of preparing a hydroxyapatite which may be used in the present invention. The hydroxyapatite in this method is prepared by the sol-gel process.

The following starting materials were used:

| | |
|---|---|
| Ca(NO₃)₂.4H₂O | mw=236.15 |
| (NH₄)₂HPO₄ | mw=132.06 |

A first solution containing 0.05M to 0.5M calcium nitrate Ca(NO₃)₂.4H₂O in dH₂O (deuterated water) was prepared. Separately a second solution containing 0.05M to 0.5M ammonium phosphate (NH₄)₂HPO₄ in dH₂O was prepared to which was added a suitable amount of an ammonia solution NH₄OH to adjust the pH of solution to about 10. The first solution was then added dropwise to the second solution. The solution was then aged for 100 hours at room temperature and a precipitate was then collected by centrifuging. The precipitate was then washed with three portions of decreasing concentrations of an ammonia solution NH₄OH and dH₂O, followed by two ethanol washes.

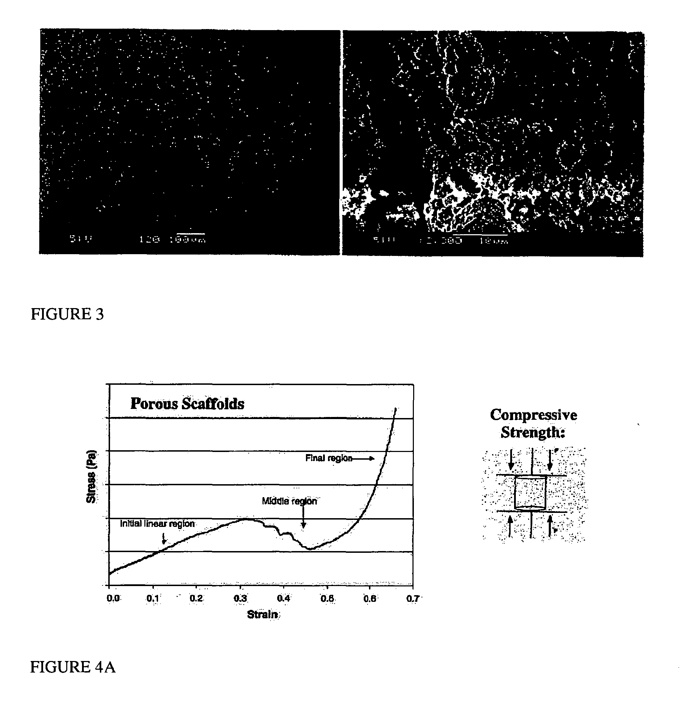

The resulting gel was air-dried for 24hrs on a watchglass, and further oven dried for 12 hrs at 120°C. The powder was ground and the resulting ground powder was then dried on a hot plate and optionally calcined at above 500°C for 5 hours. To determine the microstructure, a scanning electron micrograph of a dry hydroxyapatite produced by this method was undertaken and the results are shown in Figure 3 at resolutions of x120 and x2.3. The grains were approximately 25nm (by XRD X-ray diffraction) aggregated to form agglomerates.

### Example B

The following example is a description of a method of preparing a carbonated apatite which may be used in the present invention. The carbonated apatite in this method is prepared by the sol-gel process as follows.

The following starting materials were used:

| | |
|---|---|
| Ca(NO₃)₂.4H₂O | mw=236.15 |
| (NH₄)₂HPO₄ | mw=132.06 |
| (NH₄)HCO₃ | mw=79.06 |

A first solution containing 0.05M to 0.5M calcium nitrate Ca(NO₃)₂.4H₂O in dH₂O (deuterated water) was prepared.. Separately a second solution containing 0.05M to 0.5M ammonium phosphate (NH₄)₂HPO₄, 0.05M to 0.5M ammonium carbonate (NH₄)HCO₃ and a suitable surfactant in dH₂O was prepared. A suitable amount of an ammonia solution NH₄OH was added to adjust the pH of the solution to about 10. The first solution was then added dropwise to the second solution. The solution was then aged for 100 hours at room temperature. A precipitate was then collected by centrifuging. The precipitate was then washed with three portions of decreasing concentrations of ammonia solution NH₄OH and dH₂O, followed by two ethanol washes. ,

The resulting gel was air-dried for 24hrs on a watchglass, and further oven dried for 12 hrs at 120°C. The powder was ground and the resulting ground powder was then dried on a hot plate.

### Example 1

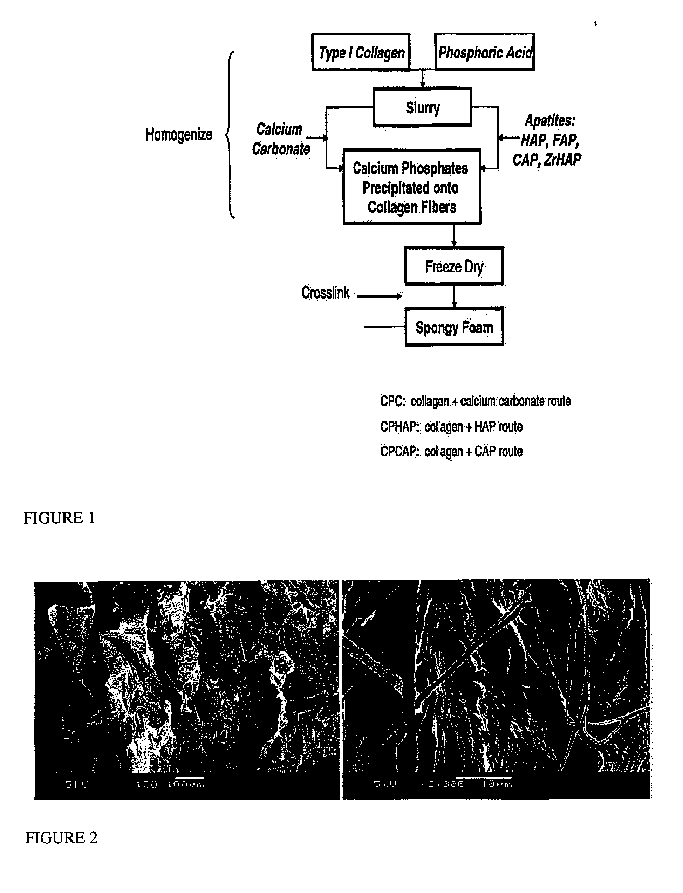

Figure 1 shows a schematic diagram for producing a scaffold in accordance with one embodiment of the present invention. In this embodiment Type 1 collagen is used such as shown in Figure 2. Figure 2 is a set of scanning electron micrographs of a dry collagen suitable for use in the present invention at resolutions of x120 and x2.3. The microstructure shows fibrils of approximately 1.5-2µm in diameter mixed with thin filmy sheets.

As shown in Figure 1, Type 1 collagen and phosphoric acid are combined into a slurry and homogenized and either calcium carbonate and/or an apatite (such as HAP, FAP, CAP, or ZrCAP) added to the slurry. The slurry is then homogenized whereby calcium phosphate and/or the apatite are interspersed and precipitated onto the collagen fibers.

The slurry is then freeze dried followed by cross-linking to form a spongy foam. The freeze-dried product may be crosslinked according to the following protocol:
1-ethyl-3-(3-dimethylaminopropyl)carbodiimide/N-hydroxysuccinimide (EDC/NHS) crosslinking_protocol - modified from the protocol of Olde Damink et al., Biomaterials 17 (1996) 765-773.

Because EDC has a molecular weight of 197 g/mol, 0.276g is used per 100ml, for example. Because NHS has a molecular weight of 115 g/mol, 0.064g is used per 100ml, for example.
(1) The freeze-dried matrices of the invention are hydrated in half the final volume of deionized water (for example hydrated in 50ml of sterile deionized water for 100ml final volume of freeze-dried product).
(2) This is followed by dissolving EDC and NHS in half the final volume of deionized water (for example dissolve in 50ml of sterile deionized water for 100ml of final volume). This solution is suitably made up fresh for each use. The final concentration is 0.014M EDC and 0.005M NHS;
(3) Sterile filter the EDC/NHS solution through a 0.2mm filter into a sterile container (or directly into a container containing the hydrated matrices). Suitably use 6mmol EDC/g collagen with an EDC:NHS ratio of 5:2;
(4) Cross-link at room temperature for about 2 hours;
(5) Discard solution as hazardous waste;
(6) Rinse matrices in sterile PBS (phosphate buffer saline solution), change to fresh, sterile PBS and incubate for about 2 hours;
(7) Rinse for about 2x10 minutes twice in sterile deionized water; and
(8) Store at 4°C for up to one week before use.

Cross-linked scaffolds were produced as described.

The effect of the following synthesis parameters on the final product were compared:
(i) Effect of freezing rate on microstructure (e.g. pore characteristics) and mechanical properties (eg compressive modulus);
(ii) Effect on inorganic powder:slurry ratio on microstructure (e.g. pore characteristics) and mechanical properties (eg compressive modulus) and chemical composition and crystalline phase;
(iii) Effect of EDC/NHS cross-linking on microstructure (e.g. pore characteristics) and mechanical properties (eg compressive modulus).

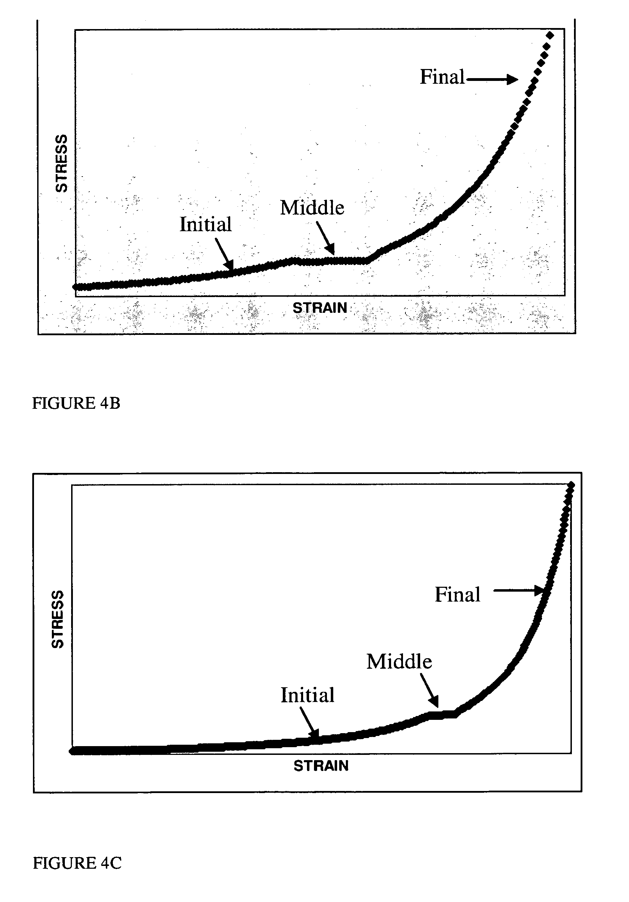

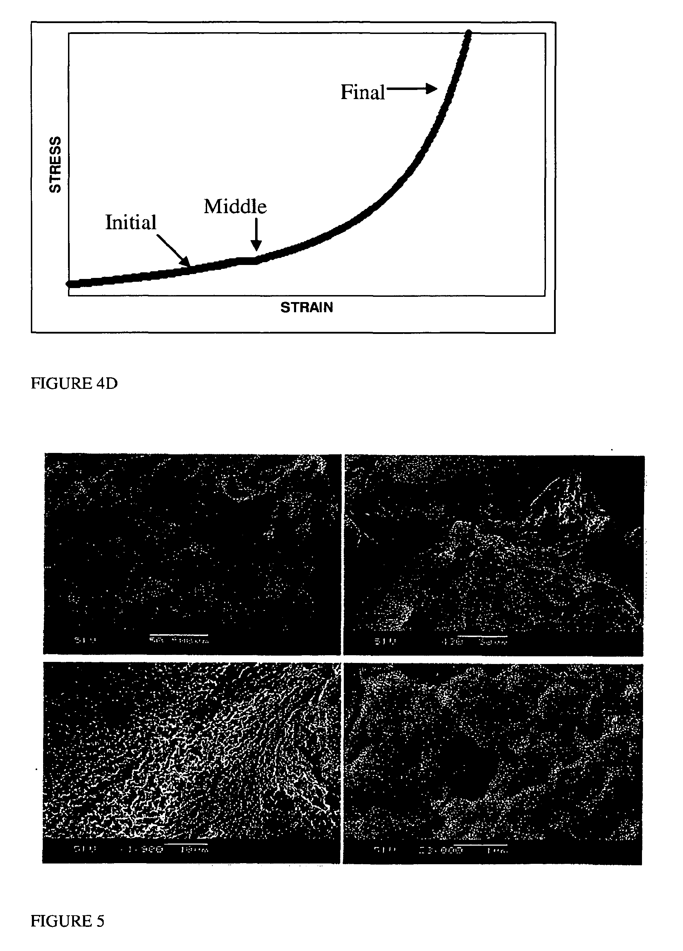

A number of collagen-inorganic scaffolds were prepared using various freezing rates, powder:slurry ratios and with optional cross-linking. Compression tests were done in Simulated Body Fluid (SBF) at 37°C according to British Standard 6039:1981 for orthopedic and dental material. Each compression point was obtained from an average of eight samples. Initial compressive modulus was obtained from compression of pores, final compressive modulus was obtained from compression of the bulk material after the pore had collapsed. Results of the compression tests are shown in Figures 4A to 4B. Figure 4A is a compression curve for a scaffold produced using 100ml of 50mM phosphoric acid, 4g collagen and 10g hydroxyapatite. Figure 4B is a compression curve for a scaffold produced using 100ml of 50mM phosphoric acid, 2g collagen and 5g carbonated apatite. Figure 4C is a compression curve for a scaffold produced using 100ml of 50mM phosphoric acid, 2g collagen and 5g hydroxyapatite. Figure 4D is a compression curve for a scaffold produced using 100ml of 100mM phosphoric acid, 2g collagen and 5g calcium carbonate. The scaffolds were frozen at -20°C.

Compression tests indicate that a slow freezing rate to a colder final freezing temperature results in larger ice crystals leading to larger pores after freeze-drying. The rate of freezing however has no apparent affect on porosity. Compression tests indicate that the final freezing temperature of -80°C leads to much lower compressive modulus for all samples compared to -20°C. It is therefore recommended that the freeze-drying temperature does not go below -50°C. Others have reported damage to the collagen structure at -80 to -50°C - Fois et al., J. Polym. Sci. Part B - Polym. Physics, 38 (7) (2000) 987-992 and this may explain the lower final compressive modulus.

It has been found that the final compressive modulus (100-300MPa) can be adjusted by varying the powder:slurry ratio. A high powder:slurry ratio results in a high initial compressive modulus: however if this ratio is too high, the collagen matrix cannot hold all the powder and excess powder would leach out during compression leading to no significant improvement or even a decease in the final compression modulus. Suitably weight ratios of power to slurry are up 5-15g powder:up to 10g collagen per 100ml of liquid solution.

It has been found the cross-linking also results in a higher compressive modulus.

### Example 2

Various micrographs of collagen-inorganic scaffolds made in accordance with the present invention were obtained and compared to that of trabecular bone.

As shown in Figure 5 micrographs of trabecular bone at various resolutions is seen to be dense, with some large pores of ~300-400µm as well as smaller pores of ~30-50µm as well as nanometer-sized pores.

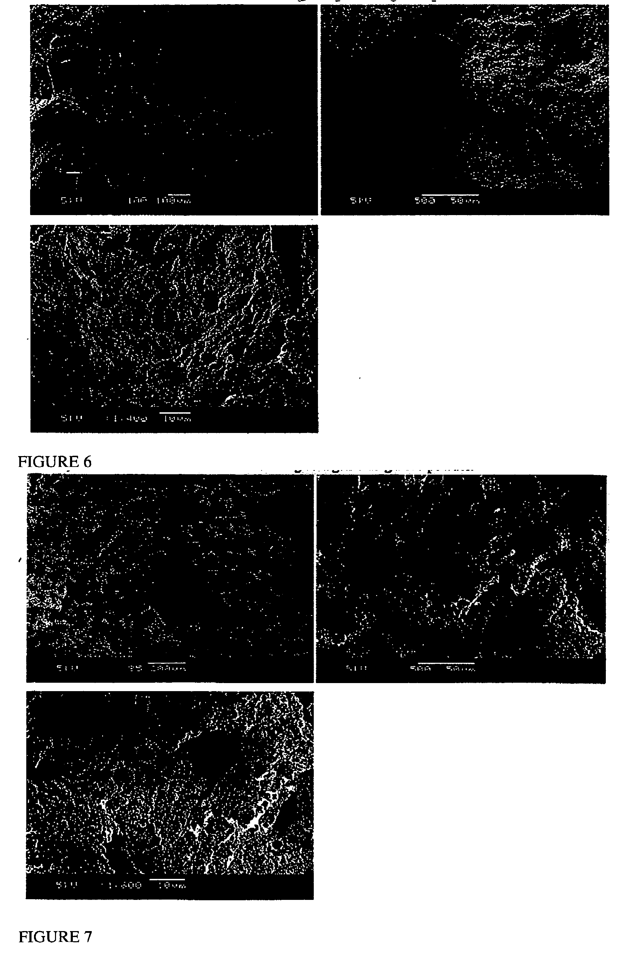

Figure 6 shows micrographs at various resolutions for a CPCAP scaffold made from 100ml of 50mM phosphoric acid, 4g collagen and 7.5g CAP (carbonated apatite powder).

It can be seen from Figure 6 that this material has the closest match with bone in microstructure, with large pores of ~200-300µm, as well as smaller pores of ~20-30µm and nanometer-sized pores from voids between CAP particles.

Figure 7 shows micrographs at various resolutions for a CPHAP scaffold made from 100ml of 50mM liquid), 4g collagen and 12g HAP (hydroxyapatite) powder using the same scaffold manufacturing method as described in Example 1. This material is bone-like but requires additional large pores of ~300µm (which can be obtained by optimizing the freezing rate), many pores of ~20-30µm as well as nanometer-sized pores from voids between HAP particles.

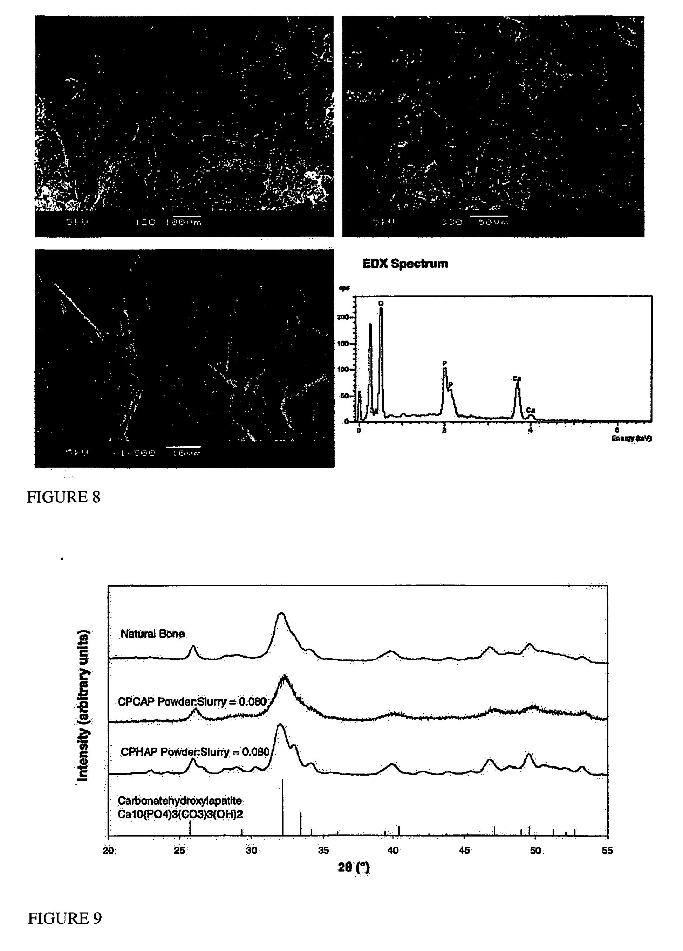

Figure 8 shows micrographs at various resolutions and an EDX (Energy Dispersive X-ray) spectrum for a CPC scaffold made from 100ml of 100mM phosphoric acid, 4g collagen and 10g calcium carbonate powder using the method described in Example 1. It can be seen that this material is bone-like but requires more large pores of ~300µm (which can be obtained by optimizing the freezing rate). The EDX spectrum shows that the particles are some form of calcium phosphate resulting from the acid-carbonate reaction.

### Example 3

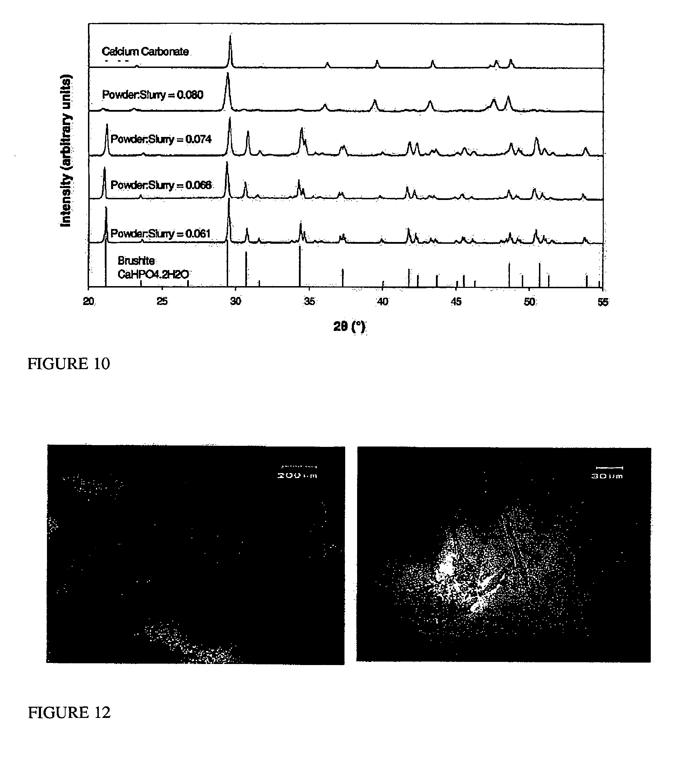

XRD and FTIR studies were undertaken of the scaffold products. Figure 9 shows XRD patterns for various collagen-carbonated apatite (CPCAP) and collagen-hydroxyapatite (CPHAP) scaffolds made using the method as described in Example 1 and that of natural bone. It is envisaged that the best match with natural bone may be obtained by combining CAP and HAP powders in optimal proportions in the same scaffold. Figure 10 shows various XRD patterns for collagen-calcium carbonate scaffolds wherein the slurry concentration was 0.6g collagen per 100mM of 100mM phosphoric acid using the general method as described in Example 1. Also shown are the XRD patterns for brushite and calcium carbonate. It can be seen that the XRD pattern varies depending on powder:slurry ratio.

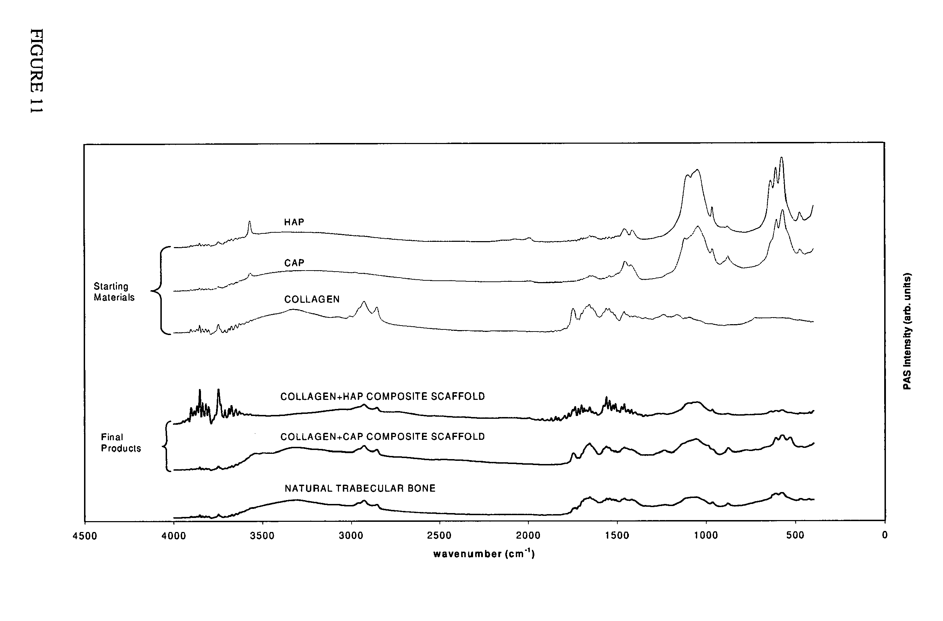

Figure 11 shows Fourier Transform Infrared spectra (FTIR) of starting materials and scaffolds made by the method of Example 1 and that of natural bone. It can be seen that the scaffolds of the invention closely match in chemical structure to natural bone.

### Example 4

*In vitro* studies with MC3T3 osteoblast cells indicate excellent cell attachment and proliferation on the material of the invention. In this example MC3T3 (mouse osteoblast) cells was cultured for one week on a scaffold of the invention using a scaffold made from 100ml of 50mM phosphoric acid, 0.6g collagen and 1g hydroxyapatite. The results are shown in Figure 12. It can be seen that cells attach to and occupy pore sites within the scaffold.

### Example 5

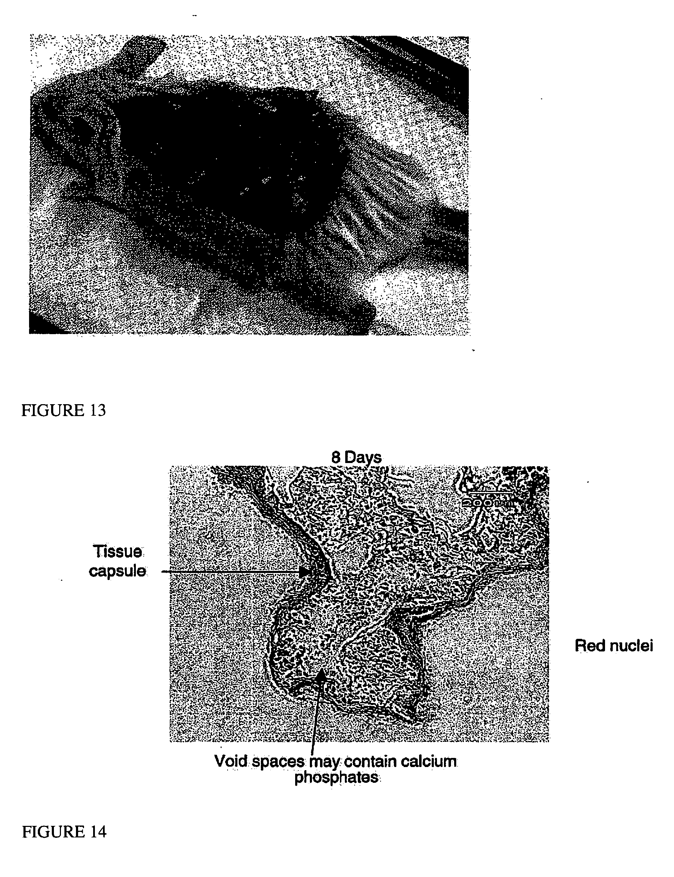

An *in vivo* study of 60 day ectopic implantation of scaffolds into SCID mice was conducted using a scaffold made from 100ml of 50mM phosphoric acid, 2g collagen and 5g hydroxyapatite. All animals survived for the full two months of experimentation and no adverse side effects were observed. The results are shown in Figure 13. This study shows the ectopic *in vivo* implantation on SCID mice shows evidence of bone, tissue and blood vessel formation in the scaffold material of the invention. The results show that the scaffolds are biocompatible, with sufficient support for tissue in growth, vascularization, osteon formation and bone mineralization. This is confirmed by the histological results shown in Figures 14 to 17.

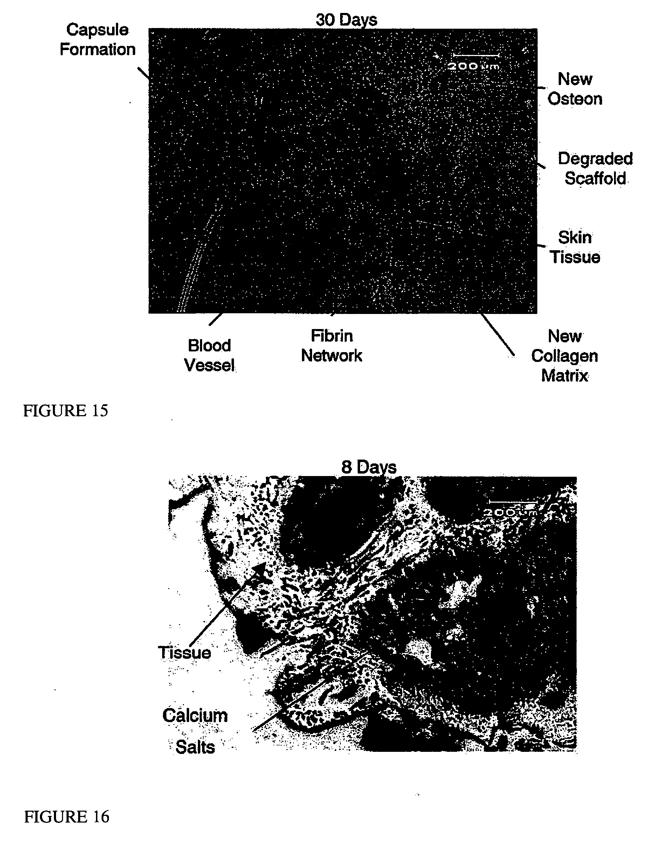

Figures 14 and 15 show Hematoxylin and Eosin staining after 8 days and 30 days. It can be seen from the Figures that tissue capsules and void spaces can be recognized which may contain calcium phosphate. In addition capsule formation, new osteon, degraded scaffold, blood vessel formation, fibrin networks, new collagen matrix and skin tissue can be seen.

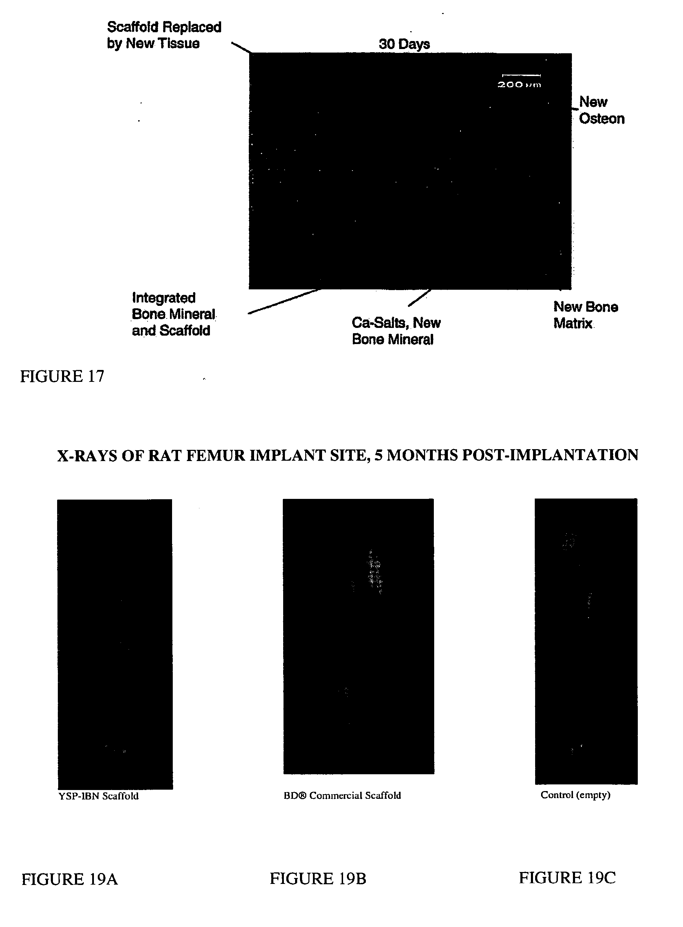

Figures 16 and 17 show Von Kossa Staining after 8 and 30 days. It can be seen from the Figures that calcium salts and tissue can be seen. The scaffold is replaced by new tissue, new osteon, new bone matrix, new bone material and integrated bone mineral and scaffold. The study shows *in vivo* implantation of the scaffold material of the invention in critical-sized defects on Wistar/SD rat femur resulted in successful healing and functioning of the defect area without the need for an external supporting cast. There was successful integration of the scaffolds with the surrounding host tissue.

### Example 6

*In vivo* load-bearing potential of porous collagen-based bone implants of the invention were determined. Porous bone implants were synthesized from collagen and calcium phosphate for bone replacement purposes.

Wistar/SD rates were chosen as this species is one of the most commonly used for *in vivo* studies of a similar nature. 50 female animals were used having an average weight of 180 to 220g. The rats used were observed for 1 week prior to surgery. Just before surgery the rats were anesthetized with Nembutal injection solution by IP (intraperitoneal) using ≤0.1ml/100g of animal weight. It was ensured that the animals were properly anesthetized by using the toe pinch test for pain reflexes. Surgery was only performed on one femur for each animal. The operating region was shaved to remove excess surface hair followed by ectopic sterilization with 70% ethanol.

The skin of the animal was cut and muscles denuded to expose the femur. The Protocol for exposing the midshaft femur was as follows:
1. Curved incision just cranial to the femoral shaft;
2. Biceps femoris (BF) encountered if incision was directly over the shaft;
3. Do not incise BF;
4. Incise fascia lata cranial to BF;
5. Retract vastus lateralis cranially;
6. Retract BF caudally; and
7. Transect femur;

A 3mm length of bone was removed from the mid-section of the femur using a surgical saw (or similar tool as appropriate) to cut completely through the femur.

Scaffolds of appropriate size were inserted in a compressed fit into the gap created by the bone removal. Scaffolds were fixed in position using stainless steel (Kirschner) pins and/or wires.

The procedure was as follows:
1. Pin inserted in retrograde fashion, exiting proximally through the trocanteric fossa;
2. Animals whip extended and the femur adducted on pushing pin through the trochanteric notch to avoid sciatic nerve and insert scaffold; and
3. Figure of Eight wire fixation used for additional support.

Muscle and skin at the trauma site was sutured shut using silk sutures (No. 4). -

After surgery, the animals were held in a common room at 22°C to recover and allowed to consume food and drink as necessary.

The animals were allowed to acclimatise (i.e. pre-operative management) for at least one week. Where necessary Buprenorphine was administered subcutaneously for post-operative pain. The animals were examined daily after surgery for about 3 weeks.

The animals were euthanized using an overdose of carbon dioxide by inhalation.

Preliminary X-rays obtained from scaffolds that were implanted for 2 months showed some bone healing with the fixation pins being removable.

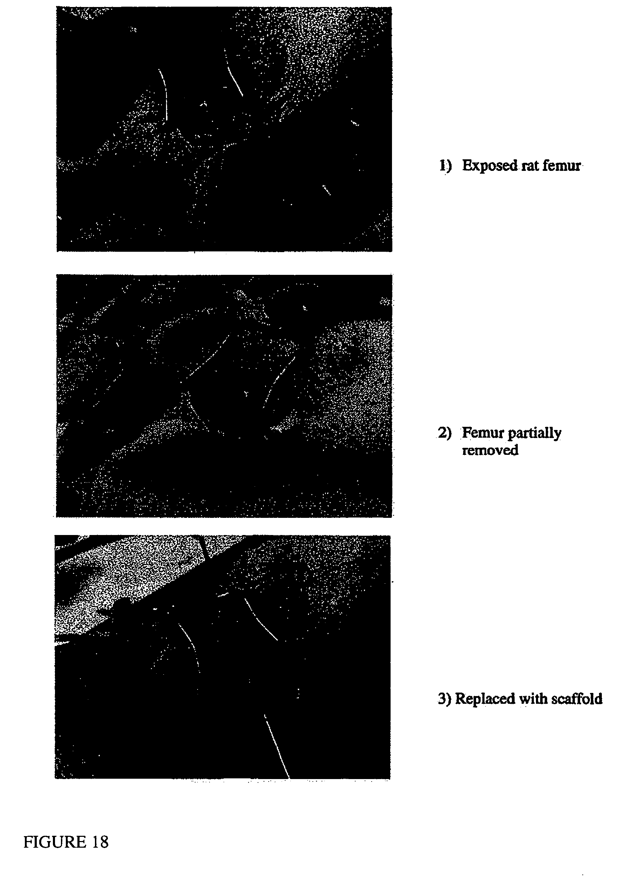

Figure 18 shows a six month implantation of a scaffold in accordance with the invention implanted by the above method into Wistar rat femur (load bearing). The results show that the scaffolds are suitable resorbable bone substitute materials to aid in healing of large fractures and bone loss. The scaffolds of the invention have sufficient strength to support the daily activities of the host animal during recovery. In addition, they demonstrate sufficient bioavailability *in vivo* for rapid attachment and proliferation.

A portion of the rats were sacrificed 5 months post-implantation and the femurs removed for X-ray. As shown in Figure 19, X-rays show that a scaffold in accordance with the present invention (collagen-hydroxyapatite marked YSP-IBN in the Figure) Figure 19A was a more successful scaffold than that of a commercial scaffold (BD Biosciences) Figure 19B compared with an empty control Figure 19C.

### Industrial Applicability

The composite of the present invention can be used in many dental, orthopedic, pharmaceutical, medical, veterinarial applications and can be used amongst others as a haemostatic agent, a scaffold for tissue repair or as a support for cell growth.

## Claims

1. A porous biomaterial-filler composite comprising a biomaterial interspersed with a filler, wherein the dry weight ratio of the biomaterial to the filler is in the range of 1:3 to 2:1, and wherein the filler is prepared by a sol-gel method to form nano-sized grains in the filler; and wherein the composite comprises:
(a) pores having a size greater than 100 micrometer;
(b) pores having a size between 30 and 50 micrometer; and
(c) small nanometer size pores.

2. The composite of claim 1, wherein the composite is for use in a method of replacing or repairing bone.

3. The composite of claim 1 or 2, wherein the composite is for use as a bone implant.

4. The composite of claim 1, wherein the composite contains at least 25 wt% (dry weight) biomaterial.

5. The composite of claim 1, wherein the composite contains 25 to 50wt% (dry weight) biomaterial with the amount of filler being between 35 to 75wt% (dry weight).

6. The composite of claim 1, wherein the composite contains 30 to 35wt% collagen and 65-70% hydroxyapatite.

7. The composite of claim 1, wherein the biomaterial is a material extracted from biological tissue selected from the group consisting of fetal tissue, skin/dermis, muscle and connective tissue.

8. The composite of claim 1, wherein the biomaterial is a biopolymer.

9. The composite of claim 1, wherein the biomaterial is selected from the group consisting of proteins, peptides, polysaccharides and other organic substance.

10. The composite of claim 1, wherein the biomaterial is selected from the group consisting of extracellular matrix proteins, fibronectin, laminin, vitronectin, tenascin, entactin, thrombospondin, elastin, gelatin, collagen, fibrillen, merosin, anchorin, chondronectin, link protein, bone sialoprotein, osteocalcin, osteopontin, epinectin, hyaluronectin, undulin, epiligrin, kalinin, proteoglycans, decorin, dermatin sulfate proteoglycans, keratin, keratin sulfate proteoglycans, aggrecan, chondroitin sulfate proteoglycans, heparin sulfate proteoglycans, biglycan, syndecan, perlecan, serglycin, glycosaminoglycans, heparin sulfate, chondroitin sulfate, dermatin sulfate, keratin sulfate, hyaluronic acid, polysaccharides, heparin, dextran sulfate, chitin, alginic acid, pectin, xylan, polyvinyl alcohol, cytokines, glycosides, glycoproteins, polypyrroles, albumin, fibrinogen, and a phospholipid.

11. The composite of claim 1, wherein the biomaterial is collagen.

12. The composite of claim 11, wherein the collagen is selected from the group consisting of collagen Type I, collagen Type II, collagen Type III, collagen Type IV, collagen Type V, collagen type VI, collagen Type VII, collagen Type VIII, collagen Type IX, collagen Type X, collagen Type XI, collagen Type XII, collagen Type XIII, collagen Type XIV, and mixtures thereof.

13. The composite of claim 12, wherein the collagen is Type 1 collagen.

14. The composite of claim 1 in the form of a foam, gel, cross-linked spongy foam, stiff foam or other construct including fibres.

15. The composite of claim 1, in the form of a scaffold.

16. The composite of claim 15 in the form of a foam cross-linked by 1-ethyl-3-(3-dimethylaminopropyl)carbodiimide/N-hydroxysuccinimide (EDC/NHS).

17. The composite of claim 15 in the form of a collagen foam cross-linked by 1-ethyl-3-(3-dimethylaminopropyl)carbodiimide/N-hydroxysuccinimide (EDC/NHS).

18. The composite of claim 1 wherein the filler comprises one or more inorganic compounds.

19. The composite of claim 18 wherein the one or more inorganic compounds is selected from the group consisting of:
(i) calcium carbonate or a calcium-containing salt;
(ii) a calcium phosphate; and
(iii) a combination of calcium carbonate and a calcium phosphate.

20. The composite of claim 19 wherein the calcium phosphate is brushite, tricalcium phosphate or octacalcium phosphate.

21. The composite of claim 1 wherein the filler is a substituted apatite or an apatite selected from the group consisting of: hydroxyapatite (HAP), fluoroapatite (FAP), carbonated apatite (CAP) and zirconium hydroxyapatite (ZrHAP).

22. The composite of claim 1 wherein the filler comprises a combination of hydroxyapatite (HAP) and carbonated apatite (CAP).

23. The composite of claim 1 wherein the filler is a carbonated apatite prepared by the sol-gel method having a grain size of between 8 to 20nm or a hydroxyapatite prepared by the sol-gel method having a grain size of between 40 to 60nm.

24. The composite of claim 1 wherein the filler is an apatite filler comprising grains having a size of from 5 to 100 nm.

25. The composite of claim 1 wherein the composite comprises by dry weight 25 to 65wt% biomaterial and 35 to 75wt% filler.

26. A method of preparing a porous biomaterial-filler composite, said composite having a dry weight ratio of biomaterial to filler in the range of 1:3 to 2:1 and comprising (a) pores having a size greater than about 100 micrometer; (b) pores having a size between about 30 and 50 micrometer; and (c) small nanometer size pores, said method comprising
combining biomaterial, a liquid and a filler to form a mixture, said filler prepared by a sol-gel method to form nano-sized grains in the filler,
homogenizing the mixture to form a slurry, and
freeze-drying the slurry to form a porous biomaterial-filler composite.

27. The method of claim 26, wherein the liquid is an acid and the amount of biomaterial in the slurry is in the range of up to about 10g biomaterial per 100ml of up to a 1000mM acid.

28. A method of preparing a porous biomaterial-filler composite, said composite having a dry weight ratio of biomaterial to filler in the range of 1:3 to 2:1 and comprising (a) pores having a size greater than about 100 micrometer; (b) pores having a size between about 30 and 50 micrometer; and (c) small nanometer size pores, said method comprising
combining biomaterial and a liquid to form a mixture,
homogenizing the mixture to form a slurry,
adding a filler to the slurry, said filler prepared by a sol-gel method to form nano-sized grains in the filler;
further homogenizing the slurry; and
freeze-drying the slurry to form a porous biomaterial-filler composite.

29. The method of claim 28, wherein the liquid is an acid and the amount of biomaterial in the slurry is in the range of up to about 10g biomaterial per 100ml of up to a 1000mM acid.

30. A method of preparing a porous biomaterial-filler composite, said composite having a dry weight ratio of biomaterial to filler in the range of 1:3 to 2:1 and comprising (a) pores having a size greater than about 100 micrometer; (b) pores having a size between about 30 and 50 micrometer; and (c) small nanometer size pores, said method comprising
combining a filler and a liquid to form a mixture, said filler prepared by a sol-gel method to form nano-sized grains in the filler,
homogenizing the mixture to form a slurry,
adding a biomaterial to the slurry;
further homogenizing the slurry; and
freeze-drying the slurry to form a porous biomaterial-filler composite.

31. The method of claim 30, wherein the liquid is an acid and the amount of biomaterial in the slurry is in the range of up to about 10g biomaterial per 100ml of up to a 1000mM acid.

## Patentansprüche

1. Poröser Biomaterial-Füllstoff-Verbundstoff, der ein Biomaterial, das mit einem Füllstoff durchsetzt ist, umfasst, worin das Trockengewichtsverhältnis zwischen dem Biomaterial und dem Füllstoff im Bereich von 1:3 bis 2:1 liegt und worin der Füllstoff durch ein Sol-Gel-Verfahren zum Bilden von nanogroßen Körnern im Füllstoff hergestellt wird; und worin der Verbundstoff Folgendes umfasst:
(a) Poren mit einer Größe von mehr als 100 µm;
(b) Poren mit einer Größe zwischen 30 und 50 µm; und
(c) Poren mit geringer Nanometergröße.

2. Verbundstoff nach Anspruch 1, worin der Verbundstoff zur Verwendung in einem Verfahren zum Ersetzen oder Reparieren von Knochen dient.

3. Verbundstoff nach Anspruch 1 oder 2, worin der Verbundstoff zur Verwendung als Knochenimplantat dient.

4. Verbundstoff nach Anspruch 1, worin der Verbundstoff zumindest 25 Gew.-% (Trockengewicht) Biomaterial enthält.

5. Verbundstoff nach Anspruch 1, worin der Verbundstoff 25 bis 50 Gew.-% (Trockengewicht) Biomaterial enthält, wobei die Menge an Füllstoff zwischen 35 und 75 Gew.-% (Trockengewicht) liegt.

6. Verbundstoff nach Anspruch 1, worin der Verbundstoff 30 bis 35 Gew.-% Kollagen und 65 bis 75 % Hydroxyapatit enthält.

7. Verbundstoff nach Anspruch 1, worin das Biomaterial ein aus aus der aus Fötengewebe, Haut/Dermis, Muskel und Bindegewebe bestehenden Gruppe ausgewähltem biologischem Gewebe extrahiertes Material ist.

8. Verbundstoff nach Anspruch 1, worin das Biomaterial ein Biopolymer ist.

9. Verbundstoff nach Anspruch 1, worin das Biomaterial aus der aus Proteinen, Peptiden, Polysacchariden und sonstiger organischer Substanz bestehenden Gruppe ausgewählt ist.

10. Verbundstoff nach Anspruch 1, worin das Biomaterial aus der aus extrazellulären Matrixproteinen, Fibronectin, Laminin, Vitronectin, Tenascin, Entactin, Thrombospondin, Elastin, Gelatine, Kollagen, Fibrillen, Merosin, Anchorin, Chondronectin, Bindeprotein, Knochensialoprotein, Osteocalcin, Osteopontin, Epinectin, Hyaluronectin, Undulin, Epiligrin, Kalinin, Proteoglykanen, Decorin, Dermatinsulfatproteoglykanen, Keratin, Keratinsulfatproteoglykanen, Aggrecan, Chondroitinsulfatproteoglykanen, Heparinsulfatproteoglykanen, Biglykan, Syndecan, Perlecan, Serglycin, Glykosaminoglykanen, Heparinsulfat, Chondroitinsulfat, Dermatinsulfat, Keratinsulfat, Hyaluronsäure, Polysacchariden, Heparin, Dextransulfat, Chitin, Alginsäure, Pectin, Xylan, Polyvinylalkohol, Zytokinen, Glykosiden, Glykoproteinen, Polypyrrolen, Albumin, Fibrinogen und einem Phospholipid bestehenden Gruppe ausgewählt ist.

11. Verbundstoff nach Anspruch 1, worin das Biomaterial Kollagen ist.

12. Verbundstoff nach Anspruch 11, worin das Kollagen aus der aus Kollagen Typ I, Kollagen Typ II, Kollagen Typ III, Kollagen Typ IV, Kollagen Typ V, Kollagen Typ VI, Kollagen Typ VII, Kollagen Typ VIII, Kollagen Typ IX, Kollagen Typ X, Kollagen Typ XI, Kollagen Typ XII, Kollagen Typ XIII, Kollagen Typ XIV und Gemischen davon bestehenden Gruppe ausgewählt ist.

13. Verbundstoff nach Anspruch 12, worin es sich bei dem Kollagen um Kollagen Typ I handelt.

14. Verbundstoff nach Anspruch 1 in Form eines Schaums, Gels, vernetzten porösen Schaums, starren Schaums oder sonstigen Fasern enthaltenden Konstrukts.

15. Verbundstoff nach Anspruch 1 in Form eines Proteingerüsts.

16. Verbundstoff nach Anspruch 15 in Form eines durch 1-Ethyl-3-(3-dimethylaminopropyl)carbodiimid/N-Hydroxysuccinimid (EDC/NHS) vernetzten Schaums.

17. Verbundstoff nach Anspruch 15 in Form eines durch 1-Ethyl-3-(3-dimethylaminopropyl)carbodiimid/N-Hydroxysuccinimid (EDC/NHS) vernetzten Kollagenschaums.

18. Verbundstoff nach Anspruch 1, worin der Füllstoff eine oder mehrere anorganische Verbindungen umfasst.

19. Verbundstoff nach Anspruch 18, worin die eine oder die mehreren anorganischen Verbindungen aus der aus
(i) Calciumcarbonat oder einem calciumhältigen Salz;
(ii) einem Calciumphosphat; und
(iii) einer Kombination aus Calciumcarbonat und einem Calciumphosphat bestehenden Gruppe ausgewählt sind.

20. Verbundstoff nach Anspruch 19, worin das Calciumphosphat Brushit, Tricalciumphosphat oder Octacalciumphosphat ist.

21. Verbundstoff nach Anspruch 1, worin der Füllstoff substituierter Apatit oder ein aus der aus Hydroxyapatit (HAP), Fluorapatit (FAP), carbonathältigem Apatit (CAP) und Zirkoniumhydroxyapatit (ZrHAP) bestehenden Gruppe ausgewählter Apatit ist.

22. Verbundstoff nach Anspruch 1, worin der Füllstoff eine Kombination aus Hydroxyapatit (HAP) und carbonathältigem Apatit (CAP) umfasst.

23. Verbundstoff nach Anspruch 1, worin der Füllstoff ein carbonathältiger Apatit, der durch ein Sol-Gel-Verfahren hergestellt wurde und eine Korngröße zwischen 8 und 20 nm aufweist, oder ein Hydroxyapatit, der durch ein Sol-Gel-Verfahren hergestellt wurde und eine Korngröße zwischen 40 und 60 nm aufweist, ist.

24. Verbundstoff nach Anspruch 1, worin der Füllstoff ein Apatitfüllstoff ist, der Körner mit einer Größe von 5 bis 100 nm umfasst.

25. Verbundstoff nach Anspruch 1, worin der Verbundstoff jeweils im Trockengewicht 25 bis 65 Gew.-% Biomaterial und 35 bis 75 Gew.-% Füllstoff umfasst.

26. Verfahren zur Herstellung eines porösen Biomaterial-Füllstoff-Verbundstoffs, wobei der Verbundstoff ein Trockengewichtsverhältnis zwischen Biomaterial und Füllstoff im Bereich von 1:3 bis 2:1 aufweist und (a) Poren mit einer Größe von mehr als 100 µm; (b) Poren mit einer Größe zwischen 30 und 50 µm; und (c) Poren mit geringer Nanometergröße umfasst, wobei das Verfahren Folgendes umfasst:
das Kombinieren von Biomaterial, einer Flüssigkeit und einem Füllstoff zu einem Gemisch, wobei der Füllstoff durch ein Sol-Gel-Verfahren zum Bilden von nanogroßen Körnern im Füllstoff hergestellt wurde,
das Homogenisieren des Gemisches, um eine Aufschlämmung zu bilden, und
das Gefriertrocknen der Aufschlämmung, um einen porösen Biomaterial-Füllstoff-Verbundstoff zu bilden.

27. Verfahren nach Anspruch 26, worin die Flüssigkeit eine Säure ist und die Menge an Biomaterial in der Aufschlämmung im Bereich von bis zu etwa 10 g Biomaterial pro 100 ml einer Säure mit bis zu 1000 mM liegt.

28. Verfahren zur Herstellung eines porösen Biomaterial-Füllstoff-Verbundstoffs, wobei der Verbundstoff ein Trockengewichtsverhältnis zwischen Biomaterial und Füllstoff im Bereich von 1:3 bis 2:1 aufweist und (a) Poren mit einer Größe von mehr als 100 µm; (b) Poren mit einer Größe zwischen 30 und 50 µm; und (c) Poren mit geringer Nanometergröße umfasst, wobei das Verfahren Folgendes umfasst:
das Kombinieren von Biomaterial und einer Flüssigkeit zu einem Gemisch,
das Homogenisieren des Gemisches, um eine Aufschlämmung zu bilden,
das Hinzufügen eines Füllstoffs zu der Aufschlämmung, wobei der Füllstoff durch ein Sol-Gel-Verfahren zum Bilden von nanogroßen Körnern im Füllstoff hergestellt wurde;
das weitere Homogenisieren der Aufschlämmung; und
das Gefriertrocknen der Aufschlämmung, um einen porösen Biomaterial-Füllstoff-Verbundstoff zu bilden.

29. Verfahren nach Anspruch 28, worin die Flüssigkeit eine Säure ist und die Menge an Biomaterial in der Aufschlämmung im Bereich von bis zu etwa 10 g Biomaterial pro 100 ml einer Säure mit bis zu 1000 mM liegt.

30. Verfahren zur Herstellung eines porösen Biomaterial-Füllstoff-Verbundstoffs, wobei der Verbundstoff ein Trockengewichtsverhältnis zwischen Biomaterial und Füllstoff im Bereich von 1:3 bis 2:1 aufweist und (a) Poren mit einer Größe von mehr als 100 µm; (b) Poren mit einer Größe zwischen 30 und 50 µm; und (c) Poren mit geringer Nanometergröße umfasst, wobei das Verfahren Folgendes umfasst:
das Kombinieren von einem Füllstoff und einer Flüssigkeit zu einem Gemisch, wobei der Füllstoff mit einem Sol-Gel-Verfahren zum Bilden von nanogroßen Körnern im Füllstoff hergestellt wurde,
das Homogenisieren des Gemisches, um eine Aufschlämmung zu bilden,
das Hinzufügen eines Biomaterials zu der Aufschlämmung;
das weitere Homogenisieren der Aufschlämmung; und
das Gefriertrocknen der Aufschlämmung, um einen porösen Biomaterial-Füllstoff-Verbundstoff zu bilden.

31. Verfahren nach Anspruch 30, worin die Flüssigkeit eine Säure ist und die Menge an Biomaterial in der Aufschlämmung im Bereich von bis zu etwa 10 g Biomaterial pro 100 ml einer Säure mit bis zu 1000 mM liegt.

## Revendications

1. Composite de matière de charge biologique poreuse qui comprend une matière biologique entrecoupée avec une matière de charge, dans lequel le rapport de poids sec entre la matière biologique et la matière de charge est de l'ordre de 1:3 à 2:1, et dans lequel la matière de charge est préparée à l'aide d'un procédé sol-gel de façon à former des nano-grains dans la matière de charge; et dans lequel le composite comprend:
(a) des pores ayant une taille supérieure à 100 micromètres;
(b) des pores ayant une taille de l'ordre de 30 à 50 micromètres; et
(c) des petits pores de taille nanométrique.

2. Composite selon la revendication 1, destiné à être utilisé avec un procédé de remplacement ou de réparation d'un os.

3. Composite selon la revendication 1 ou 2, destiné à être utilisé comme implant osseux.

4. Composite selon la revendication 1, comprenant au moins 25% en poids (poids sec) de matière biologique.

5. Composite selon la revendication 1, contenant 25 à 50% en poids (poids sec) de matière biologique, avec une quantité de matière de charge de l'ordre de 35 à 75% en poids (poids sec).

6. Composite selon la revendication 1, contenant 30 à 35% en poids de collagène et 65 à 70% d'hydroxyapatite.

7. Composite selon la revendication 1, dans lequel la matière biologique est une matière extraite d'un tissu biologique choisi parmi le groupe consistant en un tissu foetal, de la peau/du derme, un muscle, et un tissu connectif.

8. Composite selon la revendication 1, dans lequel la matière biologique est un biopolymère.

9. Composite selon la revendication 1, dans lequel la matière biologique est choisie parmi le groupe consistant en des protéines, des peptides, des polysaccharides, et d'autres substances organiques.

10. Composite selon la revendication 1, dans lequel la matière biologique est choisie parmi le groupe consistant en des protéines à matrice extracellulaire, de la fibronectine, de la laminine, de la vitronectine, de la ténascine, de l'entactine, de la thrombospondine, de l'élastine, de la gélatine, du collagène, de la fibrilline, de la mérosine, de l'anchorine, de la chondronectine, une protéine du lait, une sialoprotéine osseuse, une ostéocalcine, une ostéopontine, une épinectine, une hyaluronectine, l'unduline, l'épiligrine, la kalinine, des protéoglycanes, de la décorine, des protéoglycanes de sulfate de dermatine, de la kératine, des protéoglycanes de sulfate de kératine, de l'aggrécane, des protéoglycanes de sulfate de chondroïtine, des protéoglycanes de sulfate d'héparine, du biglycane, du syndécane, du perlécane, de la serglycine, des glycoaminoglycanes, du sulfate d'héparine, du sulfate de chondroïtine, du sulfate de dermatine, du sulfate de kératine, de l'acide hyaluronique, des polysaccharides, de l'héparine, du sulfate de dextrane, de la chitine, de l'acide alginique, de la pectine, du xylane, de l'alcool polyvinylique, des cytokines, des glycosides, des glycoprotéines, des polypyrroles, de l'albumine, du fibrinogène, et un phospholipide.

11. Composite selon la revendication 1, dans lequel la matière biologique est du collagène.

12. Composite selon la revendication 11, dans lequel le collagène est choisi parmi le groupe consistant en du collagène de type I, du collagène de type II, du collagène de type III, du collagène de type IV, du collagène de type V, du collagène de type VI, du collagène de type VII, du collagène de type VIII, du collagène de type IX, du collagène de type X, du collagène de type XI, du collagène de type XII, du collagène de type XIII, du collagène de type XIV, et des mélanges de ceux-ci.

13. Composite selon la revendication 12, dans lequel le collagène est du collagène de type I.

14. Composite selon la revendication 1 sous la forme d'une mousse, d'un gel, d'une mousse spongieuse réticulée, d'une mousse dense, ou de toute autre matière comprenant des fibres.

15. Composite selon la revendication 1, sous la forme d'un échafaudage.

16. Composite selon la revendication 15, sous la forme d'une mousse réticulée par du 1-éthyl-3-(3(diméthylaminopropyl)carbodiimide/N-hydroxysuccinimide (EDC/NHS).

17. Composite selon la revendication 15, sous la forme d'une mousse de collagène réticulée par du 1-éthyl-3-(3-diméthylaminopropyl)carbodiimide/N-hydroxysuccinimide (EDC/NHS).

18. Composite selon la revendication 1, dans lequel la matière de charge comprend un ou plusieurs composé(s) inorganique(s).

19. Composite selon la revendication 18, dans lequel ledit ou lesdits composé(s) inorganique(s) est/sont choisi(s) parmi le groupe consistant en:
(i) du carbonate de calcium ou un sel contenant du calcium;
(ii) un phosphate de calcium; et
(iii) une combinaison de carbonate de calcium et d'un phosphate de calcium.

20. Composite selon la revendication 19, dans lequel le phosphate de calcium est de la brushite, du phosphate de tricalcium ou du phosphate d'octacalcium.

21. Composite selon la revendication 1, dans lequel la matière de charge est un apatite substitué ou un apatite choisi parmi le groupe consistant en: de l'hydroxyapatite (HAP), du fluoroapatite (FAP), de l'apatite carboné (CAP) et de l'hydroxyapatite de zirconium (ZrHAP).

22. Composite selon la revendication 1, dans lequel la matière de charge comprend une combinaison d'hydroxyapatite (HAP) et d'apatite carboné (CAP).

23. Composite selon la revendication 1, dans lequel la matière de charge est un apatite carboné préparé par le procédé sol-gel et ayant une taille de grain de l'ordre de 8 à 20 nm, ou un hydroxyapatite préparé par le procédé sol-gel et ayant une taille de grain de l'ordre de 40 à 60 nm.

24. Composite selon la revendication 1, dans lequel la matière de charge est un apatite qui comprend des grains ayant une taille de l'ordre de 5 à 100 nm.

25. Composite selon la revendication 1, comprenant 25 à 65% en poids (poids sec) de matière biologique et 35 à 75% en poids (poids sec) de matière de charge.

26. Procédé de préparation d'un composite de matière de charge biologique poreuse, ledit composite ayant un rapport de poids sec entre la matière biologique et la matière de charge de l'ordre de 1:3 à 2:1, et comprenant (a) des pores ayant une taille supérieure à 100 micromètres; (b) des pores ayant une taille de l'ordre de 30 à 50 micromètres; et (c) des petits pores de taille nanométrique, ledit procédé comprenant
la combinaison de la matière biologique, d'un liquide et d'une matière de charge afin de former un mélange, ladite matière de charge étant préparée par un procédé sol-gel afin de former des grains de taille nanométrique dans la matière de charge,
l'homogénéisation du mélange afin de former une boue, et
la lyophilisation de la boue afin de former un composite de matière de charge biologique poreuse.

27. Procédé selon la revendication 26, dans lequel le liquide est un acide et la quantité de matière biologique dans la boue est de l'ordre d'environ 10 g de matière biologique pour 100 ml d'acide 1000mM.

28. Procédé de préparation d'un composite de matière de charge biologique poreuse, ledit composite ayant un rapport de poids sec entre la matière biologique et la matière de charge de l'ordre de 1:3 à 2:1, et comprenant (a) des pores ayant une taille supérieure à 100 micromètres; (b) des pores ayant une taille de l'ordre de 30 à 50 micromètres; et (c) des petits pores de taille nanométrique, ledit procédé comprenant
la combinaison de la matière biologique et d'un liquide afin de former un mélange,
l'homogénéisation du mélange afin de former une boue, l'ajout d'une matière de charge à la boue, ladite matière de charge étant préparée par un procédé sol-gel afin de former des grains de taille nanométrique dans la matière de charge;
une nouvelle homogénéisation de la boue; et
la lyophilisation de la boue afin de former un composite de matière de charge biologique poreuse.

29. Procédé selon la revendication 28, dans lequel le liquide est un acide et la quantité de matière biologique dans la boue est de l'ordre d'environ 10 g de matière biologique pour 100 ml d'acide 1000mM.

30. Procédé de préparation d'un composite de matière de charge biologique poreuse, ledit composite ayant un rapport de poids sec entre la matière biologique et la matière de charge de l'ordre 1:3 à 2:1, et comprenant (a) des pores ayant une taille supérieure à 100 micromètres; (b) des pores ayant une taille de l'ordre de 30 à 50 micromètres; et (c) des petits pores de taille nanométrique, ledit procédé comprenant
la combinaison d'une matière de charge et d'un liquide afin de former un mélange, ladite matière de charge étant préparée par un procédé sol-gel afin de former des grains de taille nanométrique dans la matière de charge,
l'homogénéisation du mélange afin de former une boue,
l'ajout d'une matière de charge à la boue;
une nouvelle homogénéisation de la boue; et
la lyophilisation de la boue afin de former un composite de matière de charge biologique poreuse.

31. Procédé selon la revendication 30, dans lequel le liquide est un acide et la quantité de matière biologique dans la boue est de l'ordre d'environ 10 g de matière biologique pour 100 ml d'acide 1000mM.
